# EUROPEAN PATENT APPLICATION

(11) **EP 3 153 591 A1**
(43) Date of publication of application: **12.04.2017**
(21) Application number: 15188569.6
(22) Date of filing: 06.10.2015
(51) Int. Cl.: C12Q 1/68

(54) **DETERMINATION OF THE RISK FOR COLORECTAL CANCER AND THE LIKELIHOOD TO SURVIVE**

(71) Applicant: Eberhard Karls Universität Tübingen, 72074 Tübingen (DE)
(72) Inventor: Weber, Alexander, 72108 Rottenburg (DE); Försti, Asta, 69115 Heidelberg (DE); Huhn, Stefanie, 69121 Heidelberg (DE)
(74) Representative: Witte, Weller & Partner Patentanwälte mbB

(57) **Abstract**

The present invention relates to a set of single-nucleotide polymorphisms (SNPs) to determine the risk of a human subject of being affected by colorectal cancer or the likelihood of surviving colorectal cancer, a method for determining the risk of a human subject of being affected by colorectal cancer or the likelihood of surviving colorectal cancer, a test kit configured for carrying out the method according to the invention, a computer program comprising program code means for causing a computer to carry out the steps of the method according to, and device configured for the implementation of this method.

## Description

The present invention relates to a set of single-nucleotide polymorphisms (SNPs) to determine the risk of a human subject of being affected by colorectal cancer or the likelihood of surviving colorectal cancer, a method for determining the risk of a human subject of being affected by colorectal cancer or the likelihood of surviving colorectal cancer, a test kit configured for carrying out the method according to the invention, a computer program comprising program code means for causing a computer to carry out the steps of the method according to, and a device configured for the implementation of this method.

Colorectal cancer (CRC), also known as colon cancer, rectal cancer or bowel cancer, is the development of cancer in the colon or rectum. It is due to the abnormal growth of cells that have the ability to invade or spread to other parts of the body. Risk factors for colorectal cancer include lifestyle, older age, and inherited genetic disorders.

Globally more than 1 million people get colorectal cancer every year resulting in about 715,000 deaths as of 2010 up from 490,000 in 1990. As of 2012 it is the second most common cause of cancer in women (9.2% of diagnoses) and the third most common in men (10.0%) with it being the fourth most common cause of cancer death after lung, stomach, and liver cancer. In Germany there are 71.000 new cases and 30.000 deaths per year. Colorectal cancer is more common in developed than developing countries. Globally incidences vary 10-fold with highest rates in Australia, New Zealand, Europe and the U.S. and lowest rates in Africa and South-Central Asia.

At present diagnosis of colorectal cancer is via sampling of areas of the colon suspicious for possible tumor development typically done during colonoscopy or sigmoidoscopy, depending on the location of the lesion. The extent of the disease is then usually determined by a CT scan of the chest, abdomen and pelvis. There are other potential imaging tests such as PET and MRI which may be used in certain cases. Colon cancer staging is done next and based on the TNM system which is determined by how much the initial tumor has spread, if and where lymph nodes are involved, and the extent of metastatic disease.

The microscopic cellular characteristics of the tumor are usually reported from the analysis of tissue taken from a biopsy or surgery. A pathology report will usually contain a description of cell type and grade. The most common colon cancer cell type is adenocarcinoma which accounts for 98% of cases. Other, rarer types include lymphoma and squamous cell carcinoma.

Current preventive strategies for colorectal cancer are based on stool tests which are widely applicable and cost-effective, however provide a high number of false-positive and -negative results. The colonoscopy, even though being an effective preventative tool that can also remove precursors of colorectal cancer is, however, only moderately accepted in the population so that only 25% of the population-at-risk (PAR) can be reached. Besides those two methods at present there is no reliable test which may allow the identification of high-risk individuals. In addition, currently there are only very few validated biomarkers allowing an assessment of therapeutic decisions.

Against this background it is an object underlying the invention to provide a reliable tool for the determination of the risk of a human subject of being affected by colorectal cancer or the likelihood of surviving colorectal cancer, by means of which the disadvantages of the methods of the prior art can be avoided or reduced. In particular a method suitable as a pre-test for determining the meaningfulness of a subsequent colonoscopy should be provided which does not require invasive surgery or which requires only a minimally invasive intervention.

Such object is solved by the use of a set of single-nucleotide polymorphisms (SNPs) to determine the risk of a human subject of being affected by colorectal cancer (CRC risk) or the likelihood of surviving colorectal cancer (CRC survival), wherein said set consists of at least two SNPs selected from one the following groups of SNPs:
'Group CRC risk': NLRP13 rs303997; NLRP2 rs1043673; NLRP3 rs35829419; NLRP6 rs6421985; NLRP8 rs306457; NLRP11 rs299163; or
'Group CRC survival': NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742.

Such object is furthermore solved by a method for determining the risk of a human subject of being affected by colorectal cancer (CRC risk) or the likelihood of surviving colorectal cancer (CRC survival), comprising the following steps:
1) Providing a biological sample of said human subject,
2) Analyzing said biological sample for single-nucleotide polymorphisms (SNPs) selected from one the following groups of SNPs:
   'Group CRC risk': NLRP13 rs303997; NLRP2 rs1043673; NLRP3 rs35829419; NLRP6 rs6421985; NLRP8 rs306457; NLRP11 rs299163; or
   'Group CRC survival': NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742;
3) Determining a CRC risk and/or a CRC survival based on the allelic status of at least two SNPs per group.

SNPs are germline nucleotide variations at a defined position in a chromosome. In the majority of the cases, and in all the SNPs pertaining to this application, these variations concern a single nucleotide. Theoretically, at each position any of the four bases can occur, i.e. A, T, G or C. According to convention, the base found in the majority of a given population is termed the major allele, where allele designates one naturally occurring variation. Per definition, should any other base occur, this variant is referred to as a "minor allele".

The inventors were able to identify two groups of SNPs which possess predictive values with respect to the risk for and survival of CRC. One group referred to as 'Group CRC risk' consists of SNPs indicative of a CRC risk, and the other group referred to 'Group CRC survival' consists of SNPs indicative of a CRC survival. Each SNP in either of these groups was found independently and significantly associated with either CRC risk ('Group CRC survival') or CRC survival ('Group CRC survival'). Importantly, based on research results the inventors realized that it is not the prevalence in the genome of an individual SNP with a specific allelic status that allows a determination on whether or not such individual has an average, increased or decreased CRC risk or CRC survival, but it is the number of SNPs having a specific allelic status out of one of the two groups. More specific, it has been found that the existence of at least two SNPs out of the 'Group CRC risk' having a specific allelic status is indicative for CRC risk, and of at least two SNPs out of the 'Group CRC survival' having a specific allelic status is indicative for CRC survival. In other words, for the predictive value with respect to CRC risk and CRC survival it is not decisive in which gene of the candidate genes the polymorphism is determined, but it is the sum of the individually CRC associated SNP alleles in the candidate genes which is decisive and which has to be ≥ 2 per group.

So far, in the art certain alleles at an individual SNP position have been identified to be individually associated with CRC risk or survival. However, the inventors found that the combined analysis for >1 SNP yielded a greater accuracy of prediction and could identify accurately patients with very high risk for developing, or dying of CRC, and patients with very low risk for developing or dying of CRC.

According to the invention "at least two" SNPs means two, three, four, five, six, seven, eight, or more SNPs.

The inventors realized that a reliable determination of the CRC risk and/or a CRC survival is possible if at least two SNPs having a specific allelic status per group can be found. However, the informative value of the method according to the invention is even higher if more than two SNPs having a specific allelic status per group can be found. E.g. the 'Group CRC risk' analysis showed a significant increase of CRC risk with increasing number of SNPs having a specific allelic status belonging to this group. Further, the 'Group CRC survival' analysis showed a significant decrease of survival time and event free survival time with an increasing number of SNPs having a specific allelic status belonging to this group.

According to the invention the "rs" or reference SNP ID number is a unique identifier for an SNP located in the indicated genes. The respective SNP is perfectly identifiable via its rs ID number in the Single Nucleotide Polymorphism Database (dbSNP) hosted by the National Center for Biotechnology Information (NCBI). For example, NLRP13 rs303997 stands for a specific nucleotide position defined by the rs ID in the gene 'NOD-like receptor family pyrin domain containing 13'. Depending on the phenotype of the examined individual this position may be e.g. occupied by the nucleobases thymine or cytosine, resulting in the amino acid arginine or glutamine in the expression product.

According to the invention "CRC risk" refers to the risk of a human subject of being affected by colorectal cancer (CRC). Such risk can e.g. be determined by the so-called "odds ratio" (OR) for a certain "group 1" of subjects - this group could, for example, be defined by the carriage of a certain allele - compared to a reference "group 2" - for example defined by the carriage of a certain other allele. The reference group is selected by the skilled person and then defined as having the value of 1. An OR > 1 means that the analyzed subject in a given "group 1" has an increased CRC risk compared to an individual in given "group 2", whereas an OR < 1 means that the analyzed subject in a given "group 1" has a decreased CRC risk compared to an individual in given "group 2".

According to the invention "CRC survival" refers to the likelihood of surviving CRC. Such likelihood can e.g. be determined via the so-called "hazard ratio" (HR) for a certain "group 1" of subjects - this group could, for example, be defined by the carriage of a certain allele - compared to a reference "group 2" - for example defined by the carriage of a certain other allele. The reference group is selected by the skilled person and then defined as having the value of 1. An HR < 1 means that the analyzed subject in a given "group 1" has a decreased risk of death due to CRC and an increased likelihood to survive CRC compared to an individual in given "group 2", whereas an HR > 1 means that the analyzed subject in a given "group 1" has an increased risk of death due to CRC and a decreased likelihood to survive CRC compared to an individual given in "group 2".

According to the invention the analysis in step (2) may be performed by any standard method configured for the determination of a specific nucleotide position in a nucleic acid or sequence thereof, respectively, such as a polymerase chain reaction (PCR) or, more specifically, an allele-specific quantitative or multiplex PCR, by mass spectrometry or by other current or future methods for determining the identity of a specific base or SNP-affected encoded amino acid at a certain nucleotide or amino acid position, respectively.

As the inventors found out, the use and method according to the invention allows a determination of the CRC risk and CRC survival for a larger target group than it is possible with the methods available in the art.

The accuracy of prediction of the use and method according to the invention may be augmented further by the incorporation of additional SNPs and their allelic statuses, respectively, or other biomarkers into each of the CRC risk or CRC survival group. It is to be understood that the scope of protection shall extend to CRC risk or CRC survival groups that includes any number of additional SNPs or biomarkers indicative of a CRC risk or a CRC survival, respectively.

Additionally, the scope shall extend to any additional stratification of risk and survival, e.g. in the case of survival overall survival (OS) or event-free survival (EFS), and dependent or independent of a certain treatment, genetic or other predisposition, ethnicity or other factor.

It goes without saying that the scope of the invention includes SNP positions that could serve as so-called "tagging" SNPs to impute the allelic status at the above listed SNP positions indirectly. "Tagging" SNPs are positions that are in the same genomic region as the SNP of interest and therefore they are usually co-inherited. The information of whether an SNP is a "tagging" SNP or not can be empirically deduced by the skilled artisan by using genetic studies. Knowledge of the allelic status of "tagging" SNP thus can be used to impute, i.e. deduce what the allelic status of the SNP of interest is.

The determination according to the invention is based on the identification of germ line variants in specific genes of the immune system, which, as this has been found by the inventors, are highly associated with CRC risk and CRC survival.

According to the invention a "biological sample" refers to any biological material containing an individual's germ line genomic information. This may be DNA - as an entire genome or fragments thereof covering at least two of above-mentioned SNPs, or respective RNA which can be reverse-transcribed for analysis. Since the identified predictive allelic differences alter the amino acid sequence, alternatively a sample containing proteins, fragments thereof or peptides which cover the corresponding coding sequence would also be included in the definition of "biological sample".

The use and method according to the invention can be embodied by a genetic test which provides results in a short period of time, e.g. in few hours or days, and which is reliable since the allele status in the sense of this invention has binary structure. While any biological sample containing germ line genomic DNA may be used as a starting material, such as stool or biopsies, such test may be minimally or non-invasive. The determination only requires a minimally or non-invasive biological sample, such as a blood or saliva sample. The method of the invention is compatible with high throughput techniques as required for routine applications in hospitals or diagnostic laboratories. It is cost-effective and needs to be applied only once per patient as it is based on the identification of germ line gene variants which are not subject to alterations during aging or the progression of the disease.

The use and method according to the invention allows a risk stratification of currently healthy individuals with respect to a decreased or increased risk of the development of CRC. Use of the invention could be made before the typical age of onset for CRC so that such stratification may result in appropriate preventative or therapeutic consequences, such as higher frequency or earlier application of preventive colonoscopic medical examinations, treatment with anti-inflammatory drugs, probiotics therapy or any other current or future preventative strategy. Furthermore, the invention allows a statement of probability on the mortality or disease progression in the context of a prevailing CRC, which may also result in improved therapeutic consequences, for example by closer monitoring of the patients, additional investigations, the application of individualized or specialized therapies or any other current or future therapeutic strategy.

In a preferred embodiment of the use and the method according to the invention each SNP indicates either an increased risk (RISK) or a reduced risk (REF) according to the following allelic status:
'Group CRC risk':

| | |
|---|---|
| NLRP13 rs303997: | thymine = RISK, cytosine = REF; |
| NLRP2 rs1043673: | adenine = RISK, cytosine = REF; |
| NLRP3 rs35829419: | cytosine = RISK, adenine = REF; |
| NLRP6 rs6421985: | thymine = RISK, guanine = REF; |
| NLRP8 rs306457: | cytosine = RISK, guanine = REF; |
| NLRP11 rs299163: | adenine = RISK, cytosine = REF; or |

'Group CRC survival':

| | |
|---|---|
| NLRP1 rs12150220: | thymine = RISK, adenine = REF; |
| NLRP2 rs1043673: | cytosine = RISK, adenine = REF; |
| NLRP5 rs10409555: | adenine = RISK, guanine = REF; |
| NLRP5 rs12462795: | guanine = RISK, cytosine = REF; |
| NLRP5 rs16986899: | cytosine = RISK, thymine = REF; |
| NLRP12 rs34436714: | cytosine = RISK, adenine = REF; |
| NLRC5 rs289723: | cytosine = RISK, adenine = REF; |
| NLRC5 rs74439742: | thymine = RISK, cytosine = REF. |

In this embodiment of the method according to the invention, the allelic status is determined and either an increased risk (RISK) or a reduced risk (REF) for each SNP is allocated as indicated above, namely in step (2.1) which takes place after step (2) and before step (3).

This embodiment of the invention allows the allocation of the risk potential to a specific allele of the respective SNP. For instance, when referring to the SNP NLRP13 rs303997 of the 'Group CRC risk' the prevalence of the nucleobase cytosine is correlated with the reference risk and is, therefore, identified as "REF" allele. The prevalence of the nucleobase thymine correlates with an increased risk (OR>1) and is, therefore, identified as "RISK" allele. When looking at the 'Group CRC survival' and the SNP NLRP1 rs12150220 of this group the prevalence of the nucleobase adenine correlates with a reference risk of death and is, therefore, identified as "REF" allele. The prevalence of the nucleobase thymine correlates with an increased risk of death and is, therefore, identified as "RISK" allele (HR>1).

In a preferred embodiment of the use and the method according to the invention for each SNP the genotype is determined as follows:
homozygous REF/REF;
heterozygous REF/RISK; or
homozygous RISK/RISK.

In this embodiment of the method according to the invention the determination of the genotype for each SNP performed as indicated above, namely in step (2.2) after step (2.1) and before step (3).

In humans the genome is diploid, each individual carries two chromosomes. Each human being has at each position in the genome a maternal and paternal allele, resulting from the maternal and the paternal chromosome transmitted via the fertilization. Therefore, at each SNP position an individual may be homozygous for the RISK allele, or for the REF allele, or may be heterozygous (one RISK, one REF allele). This results in three possible allelic forms, namely the reference alleles in homozygous form (REF/REF), the reference and risk alleles in heterozygous form (REF/RISK), and the risk alleles in homozygous form (RISK/RISK). This embodiment of the invention creates the preconditions for a more differentiated prognosis of the CRC risk or CRC survival for the examined individual. As the inventors were able to realize the determination of the respective genotype allows a more precise risk stratification of the analyzed human being.

In a preferred embodiment of the use and method according to the invention, in the latter in step (2.3), after step (2.2) and before step (3), it is concluded that:
(i) the CRC risk is:
   below average if the SNP genotype is: homozygous REF/REF;
   increased if the SNP genotype is: heterozygous REF/RISK;
   strongly increased if the SNP genotype is: homozygous RISK/RISK;
   and/or
(ii) the CRC survival is:
   above average if the SNP genotype is: homozygous REF/REF;
   reduced if the SNP genotype is: heterozygous RISK/REF;
   strongly reduced if the SNP genotype is: homozygous RISK/RISK.

This embodiment of the invention brings the skilled artisan in a position to conclude from the identified SNP genotype the diagnostic consequence in a direct and unambiguous manner. This allows a utilization of the use and method according to the invention not only by highly skilled staff but also auxiliary personnel, potentially aided by a computer program. This is one of the preconditions for the establishment of a high throughput screening.

In a preferred embodiment of the use and in the method according to the invention, in the latter in step (2.4), after step (2.3) and before step (3), the following quantifiers (q) are allocated to the respective SNP genotype:
REF/REF: q = 0;
REF/RISK: q = 1;
RISK/RISK: q = 2.

This embodiment of the use and method according to the invention allows a finer stratification of the examined individual. Further, it creates one of the prerequisite for an automated evaluation which requires numeric values for a clear classification of a measurement result into a specific risk group.

In a preferred embodiment of the use and in the method according to the invention, in the latter in step (2.5), after step (2.4) and before step (3), all q-values are summed up [∑(q)] and it is concluded that:
(i) the CRC risk is as follows:
   below average if ∑(q) = 0 to ≤ 3;
      and independently and optionally
   increased if ∑(q) > 3, preferably if ∑(q) = 4-5;
   and further independently and optionally
      strongly increased if ∑(q) > 5;
   and/or
(ii) the CRC survival is as follows:
   above average if ∑(q) = 0 to ≤ 5;
      and independently and optionally
   reduced if ∑(q) > 5, preferably if ∑(q) = 6-7;
      and further independently and optionally
   strongly reduced if ∑(q) > 7.

This embodiment of the use and method according to the invention provides an unambiguous and clear risk classification, thereby avoiding incorrect evaluation and misinterpretation. The summarizing and concluding is demonstrated in the following example: An individual to be analyzed for its risk of being affected by CRC comprises the following SNP genotypes: NLRP13 rs303997: genotype RISK/RISK → q=2, NLRP2 rs1043673: genotype RISK/REF → q=1, NLRP3 rs35829419: genotype RISK/RISK → q=2, NLRP6 rs6421985: genotype RISK/RISK → q=2, NLRP8 rs306457: genotype RISK/RISK → q=2, NLRP11 rs299163: genotype REF/REF → q=0. The sum of all quantifiers is as follows: ∑(q) = 2 + 1 + 2 + 2 + 2 + 0 = 9; this means q > 5. Conclusion: The CRC risk is strongly increased for this individual. The inventors were able to show that by combining the individual SNPs belonging to the 'Group CRC risk' to a "risk set" and the individual SNPs belonging to the 'Group CRC survival' to a "survival set" there will be an additive effect on CRC risk and survival, resulting in a twofold increase in CRC risk for carriers of ∑(q) >5 (OR >2) and a threefold worse prognosis in CRC survival for carriers of ∑(q) >7 (HR > 2.8).

The significance level of these predictions were p=5x10⁻⁴ (for CRC risk) or p=3x10⁻⁴ (for CRC survival) and thus highly statistically significant. The OR or HR values of these predictions that combine RISK vs. REF have emerged far higher than any previous report on an individual or combination of specific biomarker(s) to predict sporadic CRC risk or survival. Thus they are superior in identifying accurately most of the high risk individuals.

According to the invention "0 to ≤ 3" is to be understood as 0-1, 0-2 and/or 0-3. "0 to ≤ 5" is to be understood as 0-1, 0-2, 0-3, 0-4, and/or 0-5. "> 3" is to be understood as 4, 5, 6, 7, 8, 9, 10, ... etc. "> 5" is to be understood as 6, 7, 8, 9, 10, 11, 12, 13, ... etc. "> 7" is to be understood as 8, 9, 10, 11, 12, 13, 14, 15, ... etc.

According to the invention "independently" is to be understood as each conclusion can individually be made and form this preferred embodiment without the need of making any of the other conclusions. According to the invention "optionally" is to be understood as the respective conclusion may or may not form part of this embodiment.

In a further development of the method according to the invention said biological sample is a blood or saliva sample.

This embodiment has the advantage that such kinds of biological samples are used which provide appropriate starting material for a successful implementation of the method according to the invention. The use of a saliva sample even provides the preconditions for a non-invasive method.

In a further development of the method according to the invention said biological sample is genomic DNA.

This embodiment can be realized by the isolation of genomic DNA from the blood or saliva by standard methods which are well known to the skilled person.

Another subject-matter of the present invention is a test kit for determining the risk of a human subject of being affected by colorectal cancer (CRC risk) or the likelihood of surviving colorectal cancer (CRC survival), comprising:
- molecular probes configured for the analysis of a biological sample for single-nucleotide polymorphisms (SNPs) selected from one the following groups of SNPs:
   'Group CRC risk':
      NLRP13 rs303997; NLRP2 rs1043673; NLRP3 rs35829419; NLRP6 rs6421985; NLRP8 rs306457; NLRP11 rs299163; or
   'Group CRC survival':
      NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742,
- a manual for carrying out the method according to the invention.

Such a kit combines and provides the essential tools for carrying-out the method according to the invention. This kit results in an enormous facilitation, reduces the preparatory work and helps to avoid procedural errors in performing the method according to the invention. In particular, such kit allows the carrying out of the method of the invention even by semi-skilled personal and the implementation in routine laboratory processes.

Preferably, the "molecular probes" may be oligonucleotides, such as PCR primers or hybridization probes adapted for a use in polymerase chain reactions (PRC) or other methods known in the art for the determination of SNPs. "Molecular probes" also include peptides.

It goes without saying that such test kit may additionally comprise chemicals, reagents, buffers etc. useful or adapted for carrying out the method according to the invention.

It will be understood that the features, embodiments, further developments and advantages explained in more detail for the use and method according to the invention likewise apply to the test kit according to the invention.

Another subject-matter of the invention is a computer program comprising program code means for causing a computer to carry out the steps of the method according to the invention when said computer program is carried out on the computer.

It will be understood that the features, embodiments, further developments and advantages explained in more detail for the use and method according to the invention likewise apply to the computer program according to the invention.

Another subject-matter of the invention is a device for determining the risk of a human subject of being affected by colorectal cancer (CRC risk) or the likelihood of surviving colorectal cancer (CRC survival), comprising:
- a receiving unit for receiving a biological sample of said human subject,
- an analyzing unit configured for analyzing said biological sample for single-nucleotide polymorphisms (SNPs) selected from one the following groups of SNPs:
   'Group CRC risk':
      NLRP13 rs303997; NLRP2 rs1043673; NLRP3 rs35829419; NLRP6 rs6421985; NLRP8 rs306457; NLRP11; or
   'Group CRC survival':
      NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742; and a
- determining unit for determining a CRC risk and/or a CRC survival if at least two SNPs per group can be found.

The device may be embodied by a PCR cycler or any other appropriate apparatus. The features, embodiments, further developments and advantages explained in more detail for the use and method according to the invention likewise apply to the device according to the invention.

It will be understood that the features of the invention mentioned above and those yet to be explained below can be used not only in the respective combination indicated, but also in other combinations or in isolation, without leaving the scope of the present invention.

The invention is now further explained by means of embodiments which are only intended to illustrate the invention but not to limit its scope. Features and steps explained for a specific embodiment are to be understood as not only being applicable in the context of the respective embodiment but also in isolated position or in combination with other embodiments without departing from the scope of the invention.

Reference is made to the enclosed figures which show the following:

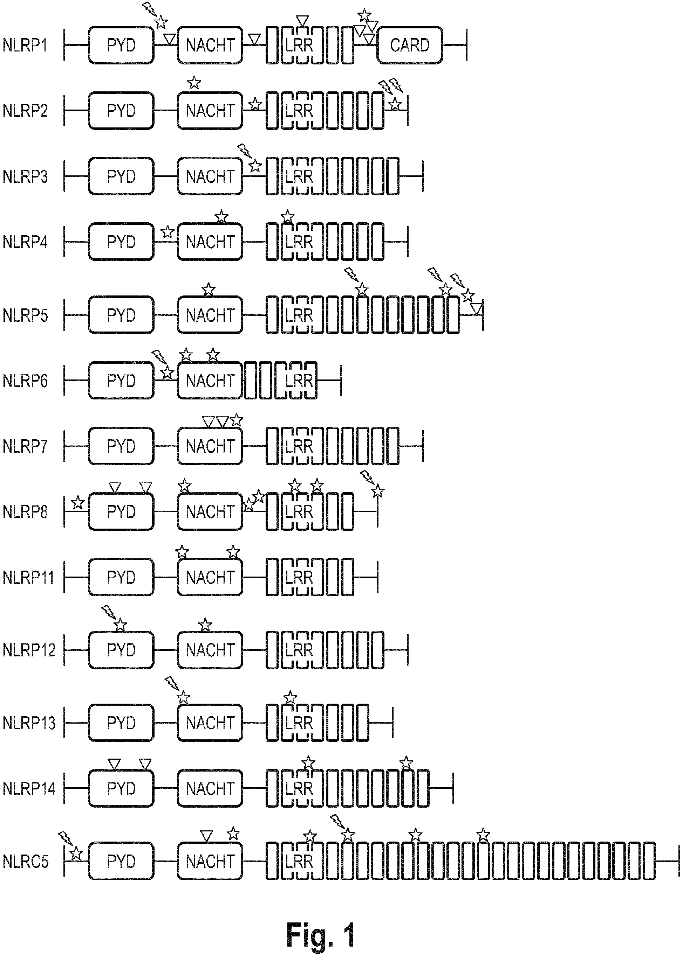

- Fig. 1: Protein structure of the candidate genes with genotyped SNPs (Star *), and all linked missense SNPs (triangle ^; r² ≥ 0.8). CRC-associated (p<0.05) SNPs are marked with lightning bolts.

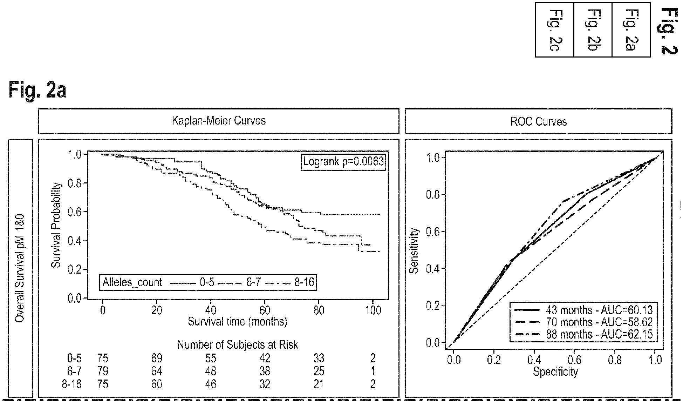

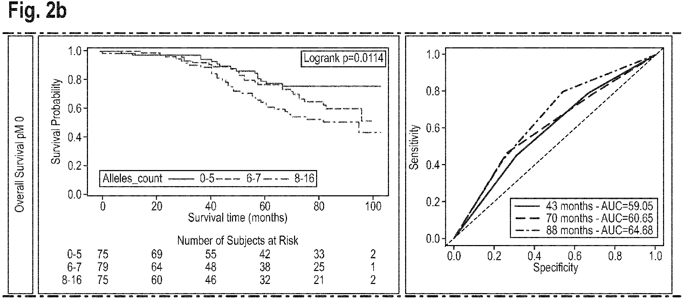

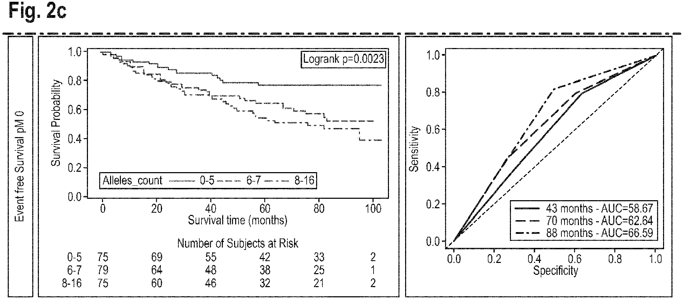

- Fig. 2: Kaplan-Meier curves with log rank p-values for the additive SNP effects on CRC survival and event free survival. SNPs with nominal association with CRC survival were assembled into a panel and patients stratified into groups of carriers for 0-5 (continuous line), 6-7 (dashed line) and 8-16 (dotted/dashed line) risk alleles. Corresponding ROC curves and AUC values for 8-16 risk alleles stratified for 43, 70 and 88 months of overall and event free survival time, respectively. pM 1: metastasis upon diagnosis; pM 0: no metastasis upon diagnosis; ROC: receiver operating characteristics curves; AUC area under the curve values. Survival SNP panel (p ≤0.05): rs12150220, rs1043673, rs10409555, rs12462795, rs16986899, rs34436714, rs289723, rs74439742.

### Embodiments

### 1. Coding variants in NOD-like receptors associated with risk and survival of colorectal cancer

### Abstract

Nod-like receptors (NLRs) are key innate pattern recognition receptors (PRRs) and regulators of inflammation. Conducting a systematic analysis of 41 non-synonymous polymorphisms (SNPs) in 21 NLR family members in a colorectal cancer (CRC) cohort (1237 cases, 787 controls) the inventors identified six SNPs associated with CRC risk and eight SNPs associated with CRC prognosis. The additive SNP effect model showed a 2-fold increase in CRC risk and a 3-fold worse prognosis for carriers of >6 risk alleles (Risk OR 2.1, p=5x10-4) or >8 risk alleles (Event-free survival HR 3.02, p=3x10-4), respectively. Expression analysis confirmed that the associated genes are indeed expressed in primary colon or rectum cells, colon adenocarcinoma and/or CRC cell lines. This provides first evidence that NLRs have a role in human CRC. The provided novel sets of genetic biomarkers may offer prediction tools for CRC risk and prognosis.

### Significance

The inventors disclose coding genetic variants of members of the Nod-like receptor (NLR) family of pattern recognition receptors (PRR) to be significantly associated with human colorectal cancer (CRC) risk or prognosis. The inventors' study for the first time provides a set of NLR genetic biomarkers that may be potentially useful in CRC prevention or clinical management.

### Introduction

Within the last few years it has become evident that in the human intestine the interplay between pattern recognition receptors (PRRs), such as Toll-like receptors (TLRs) or Nod-like receptors (NLRs), and the gut microbiota has a profound influence on the homeostasis of the human intestinal immune system and on many aspects of human health. Undisturbed and well regulated, this symbiosis is beneficial for the human host. However, a disruption of the regulatory pathways that maintain this homeostasis can result in the development of local and chronicle inflammation, inflammatory bowel disease (IBD) and colorectal cancer (CRC). The homeostasis is maintained by a physical separation of the microbial community from the gut epithelia and its sensing by PRRs, which monitor the integrity of the epithelia and the microbial community by being able to detect microbe-associated molecular patterns (MAMPs) and endogenous damage associated molecular patterns (DAMPs), respectively. The activation of PRRs such as Toll-like receptors (TLRs) or Nod-like receptors (NLR) by MAMPs or DAMPs results in the activation of multiple signalling pathways including nuclearfactor-κB (NF-κB), mitogen-activated protein kinases (MAPKs), and type I interferon (IFN) response, which then leads to the transcriptional induction of an inflammatory and anti-microbial response that includes secretory IgA, antimicrobial peptides, pyroptosis and autophagy. Some NLRs, e.g. NLRP3, additionally form so-called "inflammasome" complexes, comprised of oligomerized NLRs, the adaptor ASC and pro-caspase-1. Upon autoproteolytic cleavage, caspase-1 catalyzes the proteolytic conversion of pro-interleukin-1β and pro-interleukin-18 (IL-18) into biologically active cytokines which initiate an inflammatory and antimicrobial response.

The inventors recently reported on the involvement of TLR SNPs in CRC progression. Given the suggested concerted action of TLRs and NLRs, the connection between NLRs and CRC seemed of special interest. Provoking studies in mice and association studies in humans have suggested that defects in NLR signalling may result in inflammatory bowel disease, chronic inflammation, gastric cancer, hepatic cancer and colorectal cancer. Despite several reports for the murine system, convincing data directly connecting NLRs and human CRC are available only for NOD1, NOD2 and *NLPR3*, which were found to be associated with susceptibility, progression and treatment of spontaneous CRC, colitis and/or colitis associated colon cancer. However, in general it is unclear whether and how other NLRs contribute to human CRC. Additionally, the functional importance of NLRs that have embryonic lethal phenotypes in mice, so-called reproduction-related NLRs like NLRP2, 5 and 13, in human immunity or tumorigenesis remains enigmatic.

In order to systematically investigate the influence of functional variants in the NLR genes on CRC risk and survival, the inventors conducted a case-control study covering the majority of known non-synonymous variants (nsSNPs) in 21 genes in NLR signalling pathways. Furthermore, RNA expression of selected associated genes was measured to show the presence of the proteins in colon cells and colorectal cancer cell lines. In silico functional analysis complete the comprehensive investigation of missense SNP in the NLR gene family.

### 2. Methods and Material

### Study Population

The study was carried out on a CRC case-control population recruited in the Czech Republic. All patients (n=1237; 61.7% males) were diagnosed with colon or rectal malignancy by colonoscopy (57.8% colon) but not hereditary-nonpolyposis colorectal cancer (HNPCC). A subgroup of 477 patients was available for survival analysis with comprehensive clinical data at the time of diagnosis. Information about age, sex, TNM stage classification, grade, distant metastasis, relapse and date of death were available with a follow-up until August 31, 2011. 787 blood donors (55.4% males, cancer free at the time of sampling), were recruited as control population. Patients providing biopsy material were recruited at the University Hospital Tübingen. Healthy donors were recruited at the University Hospital Tübingen Blood transfusion unit and provided buffy coats.

According to the Helsinki declaration, all patients and blood donors provided written informed consent. The study was approved by local Ethics Committees.

### SNP Selection

21 candidate NLR genes were analysed for missense and non-sense variants using the UCSC genome browser (receptors, adaptors, and downstream binding partners, *NLRP 1-14*, *NLRC 4 and d5, NOD 1 and 2, NAIP, ASC*). Within the genomic range of the longest isoforms, 13 of the 21 genes harboured validated missense variants with a minor allele frequency (MAF) >0.01 in the CEU reference panels (Source: 1000Genomes, HapMap, dbSNP) and for potential linkage). By choosing only one SNP per linkage block (r² ≥ 0.8), 41 SNPs were selected for genotyping (Fig. 1).

### Genotyping

TaqMan SNP Genotyping Assays (Applied Biosystems; 35 SNPs) or KASP on Demand genotyping assays (LGC Genomics; 5 SNPs) were used for genotyping. Case and control samples were amplified simultaneously using the Hydrocycler 16 (LGC Genomics) (384 well format, 3ng whole genome amplified DNA extracted from blood samples). Endpoint genotype detection was carried out on the ViiA 7 Real-Time PCR System (Applied Biosystems). Call rates for 40 out of 41 SNPs were 94 - 99%. For *NLRP12* rs12610233, TaqMan and KASP assay failed to discriminate genotypes. Internal quality controls (8% randomly selected duplicates) showed a concordance rate of ≥ 99%. Samples with less than 50% call rate over all assays were excluded from the study.

### Statistical analysis

Observed genotype frequencies in controls were tested for Hardy-Weinberg equilibrium (HWE; Pearson's goodness-of-fit χ² test, deviation assumed at p<0.001). Only *NLRP11* rs12461110 was excluded for violation of HWE.

### Single variant effects on CRC risk, survival and event free survival

Odds ratios (ORs) and 95% confidence intervals (CIs) for associations between genotypes and CRC risk were estimated by logistic regression (PROC LOGISTIC, SAS V9.3; SAS Institute, Cary, NC). The estimated effects refer to the minor allele. P values were considered nominally significant at p≤0.05, with a study-wide significance level at p≤0.001 after correction for multiple testing. ORs were adjusted for the differences in the age and gender distributions between cases and controls. The estimated power was >95% for OR ≥1.5 (MAF>5%; p=0.05; dominant model).

Differences in overall survival (OS) and event free survival (EFS) between genotypes were estimated by hazard ratios (HRs) and 95% CIs using Cox regression (PROC PHREG, SAS V9.3) adjusting for age, sex, grade and stage. The estimated power was >90% for HR ≥2.0 (MAF >10%, p=0.05). OS was calculated in two models; including all patients with and without metastasis at the time of diagnosis (OS_{(pM0&1)}), and including only patients without metastasis at the time of diagnosis (OS_{(pM0)}). EFS was calculated including only patients without metastasis at the time of diagnosis (EFS₍ₚₘ₀₎). For OS, date of death or end of the study (August 31, 2011; median follow-up 58 months) was used as the end point of follow-up. For EFS in patients with non-metastatic disease at the time of diagnosis (n= 325), date of distant metastasis, tumour recurrence, death, or end of the study was used as the end point of follow-up (median follow-up 55 months). Event-free survival was defined as the time from surgery to the occurrence of distant metastasis, recurrence or death, whichever came first.

Additionally, Kaplan-Meier plots were generated and the differences between the survival functions were estimated by log rank test (PROC LIFETEST, SAS V9.3).

### Additive SNP effects on CRC risk, survival and event free survival

For all SNPs with individual significant associations (p≤0.05) the combined influence of the number of risk alleles on CRC risk and survival was estimated. The "Risk-SNP-Panel" comprised of six SNPs (max. 12 risk alleles), the "Survival-SNP-Panel" of eight SNPs (max. 16 risk alleles). OR, HRs_{OS(pM0&1)}, HRs_{OS(pM0)} and HRs_{EFS} were calculated in two steps, first individually for all existing risk allele classes of the Risk- and Survival-SNP-Panel (0-10 and 0-12 risk alleles, respectively; no individuals with >12 risk alleles), second for equally sized groups of the individual risk allele classes (Risk-SNP-Panel: 0-3/4-5/6-10, Survival-SNP-Panel 0-5/6-7/8-12). Kaplan-Meier plots were generated for the Survival-SNP-Panel and the log rank test was performed. The same analysis was conducted separately for the three *NLRP5* SNPs with significant individual results (risk alleles 0-6).

To estimate the predictive value of the Survival-SNP-Panel on CRC OS and EFS, receiver operating characteristics (ROC) curves and corresponding area under the curve (AUC) values were calculated using R (ROCR and timeROC) (adjusted for age, gender, stage and grade).

### 3. Results

### Single NLR variants are association with CRC risk, survival and event-free survival

Significant associations with CRC risk were detected for six SNPs, as depicted in the following table

**Table 1: CRC risk: genotype distribution of SNPs analysed in the Czech case-control population for SNPs with p≤0.05. Amino acid changes are given as <> with the amino acid position indicated. Data adjusted for age at diagnosis and gender; OR Odds ratio, CI confidence interval; nominal significance at p ≤ 0.05; significance level corrected for multiple testing (40 genotyped SNPs) at p ≤ 0.001.**

| **Gene** | **Risk of CRC** | | | | |
|---|---|---|---|---|---|
| **SNP** | **Genotype** | **Cases** | **Controls** | **OR (95%CI)** | **P Val** |
| **NLRP2** | C/C | 427 | 284 | 1 | |
| **rs1043673** | A/C | 574 | 355 | 1.08 (0.84-1.39) | 0.5566 |
| **1052: A<>E** | A/A | 203 | 108 | 1.41 (1.00-1.99) | **0.05** |
| | A/C + A/A | 777 | 463 | 1.16 (0.91-1.47) | 0.2312 |
| **NLRP3** | C/C | 1114 | 700 | 1 | |
| **rs35829419** | A/C | 85 | 66 | 0.63 (0.41-0.97) | **0.037** |
| **705: Q<>K** | A/A | 4 | 1 | 0.97 (0.09-10.11) | 0.9806 |
| | C/A + A/A | 89 | 67 | 0.64 (0.42-0.98) | **0.04** |
| **NLRP6** | G/G | 924 | 629 | 1 | |
| **rs6421985** | T/G | 252 | 128 | 1.36 (1.01-1.83) | **0.0421** |
| **163: L<>M** | T/T | - | - | - | - |
| | T/G + T/T | 252 | 128 | 1.36 (1.01-1.83) | **0.0421** |
| **NLRP8** | G/G | 732 | 493 | 1 | |
| **rs306457** | C/G | 415 | 235 | 1.13 (0.88-1.45) | 0.3241 |
| **1049: Ter<>Y** | C/C | 63 | 26 | 2.01 (1.09-3.72) | **0.0257** |
| | C/G + C/C | 478 | 261 | 1.20 (0.95-1.53) | 0.1267 |
| **NLRP13** | C/C | 419 | 297 | 1 | |
| **rs303997** | C/T | 564 | 334 | 1.37 (1.06-1.77) | **0.0152** |
| **247: R<>Q** | T/T | 212 | 125 | 1.54 (1.10-2.16) | **0.0128** |
| | C/T + T/T | 776 | 459 | 1.42 (1.11-1.80) | **0.0045** |
| **NLRP11** | A/A | 1070 | 662 | 1 | - |
| **rs299163** | A/C | 134 | 91 | 0.94 (0.66-1.34) | 0.7422 |
| **188: A<>S** | C/C | 7 | 12 | 0.21 (0.06-0.68) | **0.0097** |
| | A/C + C/C | 141 | 103 | 0.83 (0.59-1.17) | 0.2954 |
| **No. of** | 0-3 | 216 (32.34) | 302 (27.83) | 1 | - |
| **risk alleles** | 4-5 | 381 (57.04) | 633 (58.34) | 1.36 (1.04 - 1.79) | **0.03** |
| | 6-10 | 71 (10.63) | 150 (13.82) | 2.10 (1.38 - 3.20) | **0.0005** |

The most interesting result was found for *NLRP13* rs303997 (OR=1.42, 95%CI=1.11-1.80, P=0.0045, dominant model; adjusted for age and sex). In addition, five other SNPs showed nominally significant association (*NLRP2* rs1043673, *NLRP3* rs35829419, *NLRP6* rs6421985, *NLRP8* rs306457 and *NLRP11* rs299163). A stratification of the data according to the tumour location (colon or rectal cancer) did not reveal any significant differences in effect of the analysed variants on the risk of CRC (data not shown).

Nominally significant association with OS and/or EFS was detected for eight SNPs (data adjusted for age at diagnosis, gender, grade and stage) as depicted in the following Table 2.

**Table 2: Overall survival pM1&0 and pM0, and event free survival pM0: genotype distribution of SNPs analysed in the Czech case-control population for SNPs with p≤0.05. Amino acid changes are given as <> with the amino acid position indicated. Data adjusted for age at diagnosis and gender, grade and stage; HR Hazard ratio, CI confidence interval, nominal significance at p ≤ 0.05; significance level corrected for multiple testing (Bonferroni, 40 genotyped SNPs) at p ≤ 0.001.**

| **Gene** | | **Overall Survival (pM=1&0)** | | | | **Overall Survival (pM=0)** | | | | **Event free Survival (pM=0)** | | | |
|---|---|---|---|---|---|---|---|---|---|---|---|---|---|
| **SNP** | **Genotype** | **Cases** | **Death (%)** | **HR (95%CI)** | **p-val** | **Cases** | **Deaths (%)** | **HR (95%CI)** | **p-val** | **Cases** | **Events (%)** | **HR (95%CI)** | **p-val** |
| **NLRP1** | A/A | 106 | 46 (43.40) | 1 | | 74 | 24 (32.43) | 1 | | 74 | 29 (39.19) | 1 | |
| **rs12150220** | A/T | 177 | 86 (48.59) | 1.14 (0.79-1.63) | 0.49 | 130 | 43 (33.08) | 1.10 (0.67-1.83) | 0.7026 | 130 | 50 (38.46) | 0.95 (0.60-1.51) | 0.8187 |
| **155: H<>L** | T/T | 78 | 42 (53.85) | 1.57 (1.03-2.40) | **0.0355** | 59 | 25 (42.37) | 1.52 (0.86-2.68) | 0.1452 | 59 | 27 (45.76) | 1.31 (0.77-2.21) | 0.3206 |
| | A/T+T/T | 255 | 128 (50.20) | 1.25 (0.89-1.76) | 0.1969 | 189 | 68 (35.98) | 1.23 (0.77-1.97) | 0.3884 | 189 | 77 (40.74) | 1.05 (0.68-1.62) | 0.8211 |
| **NLRP2** | C/C | 125 | 73 (58.40) | 1 | | 87 | 38 (43.68) | 1 | | 87 | 42 (48.28) | 1 | |
| **rs1043673** | A/C | 181 | 71 (39.23) | 0.64 (0.46-0.89) | **0.0078** | 136 | 38 (27.94) | 0.59 (0.37-0.92) | **0.0213** | 136 | 45 (33.09) | 0.61 (0.40-0.93) | **0.0228** |
| **1052: A<>E** | A/A | 56 | 30 (53.57) | 0.83 (0.54-1.28) | 0.3981 | 40 | 15 (37.50) | 0.77 (0.42-1.42) | 0.4114 | 40 | 16 (40.00) | 0.75 (0.42-1.35) | 0.3416 |
| | A/C + A/A | 237 | 101 (42.62) | 0.69 (0.50-0.93) | **0.0151** | 176 | 53 (30.11) | 0.63 (0.41-0.96) | **0.0313** | 87 | 42 (48.28) | 0.64 (0.43-0.96) | **0.0288** |
| **NLRP5** | G/G | 197 | 88 (44.67) | 1 | | 143 | 42 (29.37) | 1 | | 143 | 48 (33.57) | 1 | |
| **rs10409555** | A/G | 140 | 68 (48.57) | 1.20 (0.87-1.64) | 0.2719 | 104 | 39 (37.50) | 1.53 (0.98-2.38) | 0.0623 | 104 | 47 (45.19) | 1.56 (1.04-2.35) | **0.0317** |
| **1181: V<>I** | A/A | 25 | 18 (72.00) | 1.58 (0.93-2.69) | 0.0888 | 17 | 10 (58.82) | 3.04 (1.48-6.23) | **0.0024** | 17 | 10 (58.82) | 2.36 (1.17-4.78) | **0.017** |
| | A/G + A/A | 165 | 86 (52.12) | 1.26 (0.93-1.70) | 0.1332 | 121 | 49 (40.50) | 1.69 (1.11-2.58) | **0.015** | 121 | 57 (47.11) | 1.66 (1.12-2.45) | **0.0116** |
| **NLRP5** | C/C | 268 | 122 (45.52) | 1 | | 195 | 61 (31.28) | 1 | | 195 | 69 (35.38) | 1 | |
| **rs12462795** | C/G | 90 | 47 (52.22) | 1.28 (0.91-1.81) | 0.1528 | 68 | 28 (41.18) | 1.71 (1.07-2.74) | **0.0253** | 68 | 34 (50.00) | 1.80 (1.17-2.76) | **0.0071** |
| **1108: S<>C** | G/G | 6 | 5 (83.33) | 2.79 (1.13-6.90) | **0.0261** | 3 | 2 (66.67) | 3.05 (0.73-12.74) | 0.1254 | 3 | 2 (66.67) | 2.72 (0.66-11.29) | 0.1671 |
| | C/G + G/G | 96 | 52 (54.17) | 1.36 (0.97-1.89) | 0.0714 | 71 | 30 (42.25) | 1.77 (1.12-2.81) | **0.0145** | 71 | 36 (50.70) | 1.84 (1.21-2.79) | **0.0045** |
| **NLRP5** | T/T | 253 | 118(46.64) | 1 | | 184 | 57 (30.98) | 1 | | 184 | 64 (34.78) | 1 | |
| **rs16986899** | C/T | 99 | 54 (54.55) | 1.21 (0.87-1.67) | 0.2591 | 73 | 33 (45.21) | 1.70 (1.10-2.64) | **0.0172** | 73 | 39 (53.42) | 1.74 (1.17-2.61) | **0.0068** |
| **912: M<>T** | C/C | 8 | 2 (25.00) | 0.27 (0.07-1.13) | 0.0733 | 4 | 0(0.00) | 0.00 (0.00-.) | 0.9796 | 4 | 0(0.00) | 0.00 (0.00-.) | 0.978 |
| | C/T + C/C | 107 | 56 (52.34) | 1.08 (0.78-1.49) | 0.6443 | 77 | 33 (42.86) | 1.58 (1.02-2.44) | 0.0401 | 77 | 39 (50.65) | 1.61 (1.08-2.40) | **0.0202** |
| **NLRP12** | C/C | 225 | 112(49.78) | 1 | | 161 | 60 (37.27) | 1 | | 161 | 71 (44.10) | 1 | |
| **rs34436714** | A/C | 113 | 50 (44.25) | 0.93 (0.66-1.30) | 0.6658 | 83 | 23 (27.71) | 0.72 (0.45-1.17) | 0.1898 | 83 | 26 (31.33) | 0.67 (0.43-1.06) | 0.0855 |
| **42: K<>N** | A/A | 16 | 6 (37.50) | 0.76 (0.33-1.73) | 0.511 | 13 | 3 (23.08) | 0.51 (0.16-1.65) | 0.2627 | 13 | 3 (23.08) | 0.47 (0.15-1.49) | 0.1985 |
| | A/C + A/A | 129 | 56 (43.41) | 0.91 (0.65-1.26) | 0.5542 | 96 | 26 (27.08) | 0.69 (0.43-1.10) | 0.1175 | 96 | 29 (30.21) | 0.64 (0.42-0.99) | **0.0464** |
| **NLRC5** | C/C | 192 | 101 (52.60) | 1 | | 133 | 53 (39.85) | 1 | | 133 | 61 (45.86) | 1 | |
| **rs289723** | A/C | 149 | 65 (43.62) | 0.87 (0.64-1.20) | 0.3999 | 113 | 33 (29.20) | 0.72 (0.46-1.11) | 0.1386 | 113 | 37 (32.74) | 0.61 (0.41-0.93) | **0.0208** |
| **1105: Q<>K** | A/A | 27 | 11 (40.74) | 0.99 (0.53-1.86) | 0.9777 | 23 | 8 (34.78) | 1.13 (0.53-2.41) | 0.7449 | 23 | 10 (43.48) | 1.04 (0.53-2.04) | 0.9179 |
| | A/C + A/A | 176 | 76 (43.18) | 0.89 (0.66-1.20) | 0.4417 | 136 | 41 (30.15) | 0.77 (0.51-1.17) | 0.2194 | 136 | 47 (34.56) | 0.67 (0.46-0.99) | **0.044** |
| **NLRC5** | C/C | 282 | 128(45.39) | 1 | | 204 | 64 (31.37) | 1 | | 204 | 73 (35.78) | 1 | |
| **rs74439742** | C/T | 75 | 43 (57.33) | 1.42 (1.00-2.00) | **0.0501** | 56 | 26 (46.43) | 1.67 (1.05-2.65) | **0.0304** | 56 | 31 (55.36) | 1.63 (1.06-2.49) | **0.0262** |
| **191: P<>L** | T/T | 10 | 7 (70.00) | 2.39 (1.10-5.19) | **0.0272** | 7 | 4(57.14) | 3.07 (1.07-8.80) | **0.0368** | 7 | 4 (57.14) | 2.71 (0.96-7.67) | 0.061 |
| | C/T + T/T | 85 | 50 (58.82) | 1.50 (1.08-2.09) | **0.0157** | 63 | 30 (47.62) | 1.77 (1.13-2.75) | **0.0121** | 63 | 35 (55.56) | 1.70 (1.13-2.57) | **0.0116** |
| **No. of** | 0-5 | 97 | 34 (35.05) | 1 | **-** | 75 | 15 (20.00) | 1 | **-** | 75 | 16 (21.33) | 1 | |
| **risk alleles** | 6-7 | 110 | 52 (47.27) | 1.43 (0.92 - 2.21) | 0.11 | 79 | 26 (32.91) | 1.53 (0.81 - 2.90) | 0.19 | 79 | 32 (40.51) | 2.06 (1.12 - 3.77) | **0.02** |
| | 8-12 | 108 | 62 (57.41) | 1.88 (1.23 - 2.86) | **0.003** | 75 | 33 (44.00) | 2.89 (1.55 - 5.37) | **0.0008** | 75 | 37(49.33) | 3.02 (1.66 - 5.48) | **0.0003** |

Strikingly, three unlinked SNPs in *NLPR5* (r²<0.5) were associated with shorter OS_{(pM0)} and EFS_{(pM0)} in carriers of the minor allele (rs10409555: HR_{OS(pM0)}=1.69, p=0.015, HR_{EFS(pM0)}=1.66, p=0.012; rs12462795: HR_{OS(pM0)}=1.77, p=0.0145, HR_{EFS(pM0)} =1.84, p=0.0045; rs16986899: HR_{OS(pM0)}=1.58, p=0.04, HR_{EFS(pM0)} =1.61, p=0.02). In addition, five other variants in three different NLR genes showed nominal significance with OS or EFS (*NLRP1* rs12150220; *NLRP2* rs1043673; *NLRP12* rs34436714; *NLRC5* rs289723; *NLRC5* rs74439742). Thus, individual NLR risk alleles appear to influence CRC risk or progression, respectively.

### NLR variants have an additive effect on CRC risk and survival

To investigate, if carriage of multiple risk alleles has an additive effect on risk or survival, a combined analyses of the additive effect of the five SNPs individually associated with CRC risk (Risk-SNP-Panel; p≤0.05) eight SNPs individually associated with CRC OS and EFS (Survival-SNP-Panel; p≤0.05) was conducted. The Risk-SNP-Panel analysis showed a significant increase of CRC risk with increasing number of risk allele, with a maximum risk for carriers of 6-10 risk alleles (OR 1.9, p=0.0005; Table 2). The association remained significant after correction for the multiple testing. A likelihood ratio test, comparing a risk model including only age and gender with a risk model including age, gender and the Risk-SNP-Panel, indicated a significant effect of the Risk-SNP-Panel on CRC risk (p=0.002).

The Survival-SNP-Panel analysis showed a significant decrease of survival time and event free survival time with an increasing number of risk alleles. The maximum effect was detected for carriers of 8-12 risk alleles (HR_{OS(pM0&1)} 1.88, p=0.003; HR_{OS(pM0)} 2.89 p=0,0008 and HR_{EFS} 3.02, p=0.0003, respectively; Table 2). The association with HR_{OS(pM0)} and HR_{EFS} remained significant after correction for the multiple testing. The results of the Kaplan-Meier analysis for the Survival SNP Panel are concordant to the results obtained in the HR analysis. Kaplan-Meier plots showed significant differences between the survival functions among the groups with different numbers of risk alleles (log rank test: OS_{(pM0&1)} p=0.0063, OS_{(pM0)} p=0.0114, EFS p=0.0023, Figure 2), indicating a decreased survival rate for carriers of equal or more than eight risk alleles. The AUC values, estimating the predictive value of the Survival-SNP-Panel on OS and EFS of CRC, show the strongest predictive value for long term EFS (88 month AUC 66.6) (Figure 2).

In *NLRP5,* associations of three individual unlinked SNPs with CRC survival were detected. To analyse the magnitude of impact of the three *NLRP5* SNPs on the Survival-SNP-Panel, a NLRP5-SNP-Panel was calculated. This analysis showed that the three variants in *NLRP5* are the main contributors to the association (HR_{OS(pM0)} 1.86 (1-3 risk alleles; p=0.01); HR_{OS(pM0)} 2.45 (4-6 risk alleles; p=0.005; HR_{EFS} 1.77 (1-3 risk alleles; p=0.01) HR_{EFS} 2.59 (4-6 risk alleles; p=0.001)). The association with EFS remained significant after correction for the multiple testing and the results of the Kaplan-Meier analysis are concordant to the results obtained in the HR analysis (log rank test: OS_{(pM0)} p=0.05, EFS p=0.01; Data not shown).

In conclusion the inventors' results indicate that the synergetic system of NLRs might be affected by several co-occurring genetic variants, resulting in additive effects on development and progression.

### In silico analysis of indicates functional relevance and conservation

To gain a first insight into a possible functional involvement, all SNPs were mapped to their location in the protein. Of the 12 SNP of the Risk- and Survival-SNP-Panel only three variants are located in NLR protein domains (Figure 1), *NLPR5* rs16986899 and in the LRR domain, *NLPR12* rs34436714 in the PYD domain, and *NLRP13* rs303997 in the NACHT domain of the proteins. Other SNPs (e.g. NLRP6 rs6421985) map to linker regions. In case of NLRC4, the linker regions may be functionally important and the subject of post-translational regulation. All but two SNP (*NLRP5* rs10409555 and rs16986899) are predicted to be deleterious or damaging, and/or are predicted to result in non-sense mediated decay or retained introns (SIFT & PolyPhen).

As confirmed by multiple sequence alignments, 9/11 of the CRC-associated coding SNPs either alter a highly conserved amino acid to unique new amino acid *(NLRP1* rs12150220 H155L, *NLRP5* rs12462795 S1108C, *NLRC5* rs289723 Q1105K) or affect a less conserved amino acid where the risk allele is unique for the human lineage (*NLRP2* rs1033673 A1052E, *NLRP3* rs35829419 Q705K, *NLRP6* rs6421985 L163M, *NLRP12* rs34436714 K42N, *NLRP13* rs303997 Q247R, *NLRC5* rs74439742 P191 L). The level of conservation of the SNP locations may indicate relevance for NLR protein function.

### 4. Summary

The inventors' systematic investigation of known missense SNPs in 21 NLR genes provides first evidence that multiple NLRs other than NLRP3 may have a role in human CRC. Out of 41 tested missense SNPs, six were significantly associated with CRC risk, eight with CRC survival. The inventors' candidate gene study is using a genetically homogeneous, clinically well-defined study population with a sufficient power to detect OR down to 1.5. For the survival analysis only newly diagnosed CRC cases with nearly complete clinical data were included to the study, allowing for the evaluation of the SNPs as independent prognostic markers. However, this restriction decreased the power of the study to HR ≥2.0.

Most significantly, the inventors were able to show that by combining the individual associated SNP to a "Risk-SNP-Panel" and a "Survival-SNP-Panel", NLR missense SNPs have an additive effect on CRC risk and survival, resulting in a twofold increase in CRC risk and a threefold worse prognosis for carriers of more than six and more than eight risk alleles, respectively, on a study wide significance level of P<0.001 (CRC risk: >6 risk alleles OR 2.1, P value 5x10-4; event free survival: >8 risk alleles OR 3.02, P value 3x10-4). Both SNP-Panels provide novel sets of genetic biomarkers which may offer prediction tools for CRC risk and progression. Whereas rare germline mutations such as *APC, MLH1* or *MSH2* have clinical for hereditary CRC, up to date, no biomarker panel for CRC risk prediction is established for non-syndromic CRC. Furthermore, biomarker panels for survival prediction are rare and not routinely used in clinical practice either due to the low predictive value of the biomarkers, or due to contradictory study results. Comparing the inventors' Survival-SNP-Panel to a panel suggested by Zlobec et al. (2008), Multimarker phenotype predicts adverse survival in patients with lymph node-negative colorectal cancer. Cancer. 2008;112:495-502, the inventors' Survival-SNP-Panel preforms better in terms of HR and p-values, equally good in terms of AUC values and outperforms the published study in terms of power and study size. Of note, the high OR and HR in the SNP-Panels did not concern only a small percentage of highly predisposed carriers but implicated up to 30% of the CRC cases in a high risk group for disease progression in our study population. The inventors propose that the presented or modified (e.g. by incorporating additional PRR SNPs) "SNP-Panels" for risk and survival prediction can provide a fast, unambiguous and less costly method for risk and outcome prediction than e.g. expression-based biomarker panels or traditional preventative screening by detection of occult blood or by colonoscopy, and - since genetic analysis can be conducted at any age - would provide a means for identifying high-risk individuals long before cancer development.

In conclusion, the inventors' results illustrate the importance of NLRs and the intestinal immune system in CRC development and most interestingly, CRC survival. Future studies of these variants and other affected genes in murine models and human CRC may contribute to uncover the still poorly understood role of NLRs within the intestinal immune system and in CRC development and survival and may corroborate the value "PRR SNP-Panels" as the biomarkers in CRC.

## Claims

1. Use of a set of single-nucleotide polymorphisms (SNPs) to determine the risk of a human subject of being affected by colorectal cancer (CRC risk) or the likelihood of surviving colorectal cancer (CRC survival), wherein said set consists of at least two SNPs selected from one the following groups of SNPs:
'Group CRC risk':
NLRP13 rs303997; NLRP2 rs1043673; NLRP3 rs35829419; NLRP6 rs6421985; NLRP8 rs306457; NLRP11 rs299163; or
'Group CRC survival':
NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742.

2. Use of claim 1, wherein each SNP indicates either an increased risk (RISK) or a reduced risk (REF) according to the following allelic status:
'Group CRC risk':
| | |
|---|---|
| NLRP13 rs303997: | thymine = RISK, cytosine = REF; |
| NLRP2 rs1043673: | adenine = RISK, cytosine = REF; |
| NLRP3 rs35829419: | cytosine = RISK, adenine = REF; |
| NLRP6 rs6421985: | thymine = RISK, guanine = REF; |
| NLRP8 rs306457: | cytosine = RISK, guanine = REF; |
| NLRP11 rs299163: | adenine = RISK, cytosine = REF; or |
'Group CRC survival':
| | |
|---|---|
| NLRP1 rs12150220: | thymine = RISK, adenine = REF; |
| NLRP2 rs1043673: | cytosine = RISK, adenine = REF; |
| NLRP5 rs10409555: | adenine = RISK, guanine = REF; |
| NLRP5 rs12462795: | guanine = RISK, cytosine = REF; |
| NLRP5 rs16986899: | cytosine = RISK, thymine = REF; |
| NLRP12 rs34436714: | cytosine = RISK, adenine = REF; |
| NLRC5 rs289723: | cytosine = RISK, adenine = REF; |
| NLRC5 rs74439742: | thymine = RISK, cytosine = REF. |

3. Use of claim 2, wherein for each SNP the genotype is determined as follows:
homozygous REF/REF;
heterozygous REF/RISK; or
homozygous RISK/RISK.

4. Use of claim 3, wherein from the genotype of the SNPs it is concluded that:
(i) the CRC risk is:
below average if the SNP genotype is: homozygous REF/REF;
increased if the SNP genotype is: heterozygous REF/RISK;
strongly increased if the SNP genotype is: homozygous RISK/RISK;
and/or
(ii) the CRC survival is:
above average if the SNP genotype is: homozygous REF/REF;
reduced if the SNP genotype is: heterozygous RISK/REF;
strongly reduced if the SNP genotype is: homozygous RISK/RISK.

5. Use of any of the preceding claims, wherein the following quantifiers (q) are allocated to the respective SNP genotype:
REF/REF: q = 0;
REF/RISK: q = 1;
RISK/RISK: q = 2.

6. Use of claim 5, wherein all q-values are summed up [∑(q)] and it is concluded that:
(i) the CRC risk is as follows:
below average if ∑(q) = 0 to ≤ 3;
and independently and optionally
increased if ∑(q) > 3, preferably if ∑(q) = 4-5;
and further independently and optionally
strongly increased if ∑(q) > 5;
and/or
(ii) the CRC survival is as follows:
above average if ∑(q) = 0 to ≤ 5;
and independently and optionally
reduced if ∑(q) > 5, preferably if ∑(q) = 6-7;
and further independently and optionally
strongly reduced if ∑(q) > 7.

7. Method for determining the risk of a human subject of being affected by colorectal cancer (CRC risk) or the likelihood of surviving colorectal cancer (CRC survival), comprising the following steps:
1) Providing a biological sample of said human subject,
2) Analyzing said biological sample for single-nucleotide polymorphisms (SNPs) selected from one the following groups of SNPs:
'Group CRC risk':
NLRP13 rs303997; NLRP2 rs1043673; NLRP3 rs35829419; NLRP6 rs6421985; NLRP8 rs306457; NLRP11 rs299163; or
'Group CRC survival':
NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742;
3) Determining a CRC risk and/or a CRC survival based on the allelic status of at least two SNPs per group.

8. Method of claim 7, wherein after step (2) and before step (3) the following step is performed:
2.1 determining the allelic status and allocating either an increased risk (RISK) or a reduced risk (REF) for each SNP as follows:
'Group CRC risk':
| | |
|---|---|
| NLRP13 rs303997: | thymine = RISK, cytosine = REF; |
| NLRP2 rs1043673: | adenine = RISK, cytosine = REF; |
| NLRP3 rs35829419: | cytosine = RISK, adenine = REF; |
| NLRP6 rs6421985: | thymine = RISK, guanine = REF; |
| NLRP8 rs306457: | cytosine = RISK, guanine = REF; |
| NLRP11 rs299163: | adenine = RISK, cytosine = REF; or |
'Group CRC survival':
| | |
|---|---|
| NLRP1 rs12150220: | thymine = RISK, adenine = REF; |
| NLRP2 rs1043673: | cytosine = RISK, adenine = REF; |
| NLRP5 rs10409555: | adenine = RISK, guanine = REF; |
| NLRP5 rs12462795: | guanine = RISK, cytosine = REF; |
| NLRP5 rs16986899: | cytosine = RISK, thymine = REF; |
| NLRP12 rs34436714: | cytosine = RISK, adenine = REF; |
| NLRC5 rs289723: | cytosine = RISK, adenine = REF; |
| NLRC5 rs74439742: | thymine = RISK, cytosine = REF. |

9. The method of claim 7 or 8, wherein after step (2.1) and before step (3) the following step is performed:
2.2 determining the genotype for each SNP as follows:
homozygous REF/REF;
heterozygous REF/RISK; or
homozygous RISK/RISK.

10. The method of claim 9, wherein after step (2.2) and before step (3) the following step is performed:
2.3 allocating the following quantifiers (q) to the respective SNP genotype as follows:
REF/REF: q = 0;
REF/RISK: q = 1;
RISK/RISK: q = 2.

11. The method of claim 9, wherein after step (2.3) and before step (3) the following step is performed:
2.4 summarizing all q-values [∑(q)] and concluding that:
(i) the CRC risk is as follows:
below average if ∑(q) = 0 to ≤ 3;
and independently and optionally
increased if ∑(q) > 3, preferably if ∑(q) = 4-5;
and further independently and optionally
strongly increased if ∑(q) > 5;
and/or
(ii) the CRC survival is as follows:
above average if ∑(q) = 0 to ≤ 5;
and independently and optionally
reduced if ∑(q) > 5, preferably if ∑(q) = 6-7;
and further independently and optionally
strongly reduced if ∑(q) > 7.

12. Method of any of claims 7-11, wherein said biological sample is a blood or saliva sample.

13. Method of any of claims 7-12, wherein said biological sample is genomic DNA.

14. Test kit for determining the risk of a human subject of being affected by colorectal cancer (CRC risk) or the likelihood of surviving colorectal cancer (CRC survival), comprising:
- molecular probes, preferably oligonucleotides, configured for the analysis of a biological sample for single-nucleotide polymorphisms (SNPs) selected from one the following groups of SNPs:
'Group CRC risk':
NLRP13 rs303997; NLRP2 rs1043673; NLRP3 rs35829419; NLRP6 rs6421985; NLRP8 rs306457; NLRP11 rs299163; or
'Group CRC survival':
NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742,
- a manual for carrying out the method according to any of claims 7-13.

15. Computer program comprising program code means for causing a computer to carry out the steps of the method according to any of claims 7 to 13 when said computer program is carried out on the computer

16. Device for determining the risk of a human subject of being affected by colorectal cancer (CRC risk) or the likelihood of surviving colorectal cancer (CRC survival), comprising:
- a receiving unit for receiving a biological sample of said human subject,
- an analyzing unit configured for analyzing said biological sample for single-nucleotide polymorphisms (SNPs) selected from one the following groups of SNPs:
'Group CRC risk':
NLRP13 rs303997; NLRP2 rs1043673; NLRP3 rs35829419; NLRP6 rs6421985; NLRP8 rs306457; NLRP11; or
'Group CRC survival':
NLRP1 rs12150220; NLRP2 rs1043673; NLRP5 rs10409555; NLRP5 rs12462795; NLRP5 rs16986899; NLRP12 rs34436714; NLRC5 rs289723; NLRC5 rs74439742; and a
- determining unit for determining a CRC risk and/or a CRC survival if at least two SNPs per group can be found.
